# Europäisches Patentamt

# European Patent Office

# Office européen des brevets

(11) Veröffentlichungsnummer : **0 423 526 B1**

(12) **EUROPÄISCHE PATENTSCHRIFT**

(45) Veröffentlichungstag der Patentschrift :
18.11.93 Patentblatt 93/46

(51) Int. Cl.⁵ : **C07C 209/22**, C07D 295/02

(21) Anmeldenummer : 90118755.9

(22) Anmeldetag : 29.09.90

(54) **Verfahren zur Herstellung von Triethylendiamin und Piperazin.**

(30) Priorität : 14.10.89 DE 3934459

(43) Veröffentlichungstag der Anmeldung :
24.04.91 Patentblatt 91/17

(45) Bekanntmachung des Hinweises auf die
Patenterteilung :
18.11.93 Patentblatt 93/46

(84) Benannte Vertragsstaaten :
BE DE FR NL SE

(56) Entgegenhaltungen :
EP-A- 0 010 671
EP-A- 0 312 734
EP-A- 0 313 753
WO-A-89/06229
DE-A- 1 745 627
DE-A- 3 543 228
DE-T- 3 690 621
JOURNAL OF PHYSICAL CHEMISTRY, vol. 89,
no. 9, 25 April 1985, EASTON, US, Seiten 1569 -
1571; C. T-W. CHU et al: "ISOMORPHOUS
SUBSTITUTION IN ZEOLITE FRAMEWORKS."
J. Chem. Soc., Farad. Trans. (1), 83 (1987),
487-494

(73) Patentinhaber : BAYER AG
D-51368 Leverkusen (DE)

(72) Erfinder : Weitkamp, Jens, Prof. Dr.
Rotkehlchenweg
D-2900 Oldenburg (DE)
Erfinder : Ernst, Stefan, Dr.
Winkelweg 16
D-2900 Oldenburg (DE)
Erfinder : Lindner, Dieter
Apenrader Strasse 21
D-2900 Oldenburg (DE)
Erfinder : Buysch, Hans-Josef, Dr.
Brandenburger Strasse 28
D-4150 Krefeld (DE)
Erfinder : Botta, Artur, Dr.
Bodelschwinghstrasse 119
D-4150 Krefeld (DE)
Erfinder : Puppe, Lothar, Dr.
Am Weiher 10A
D-5093 Burscheid (DE)

**Beschreibung**

Die vorliegende Erfindung betrifft ein Verfahren zur Herstellung von Triethylendiamin (TEDA) und Piperazin (PA) aus einem Ethylendiamin (EDA)-Wasser-Gemisch, die durch Zeolithe vom Pentasil-Typ mit abgeschwächter Acidität katalysiert wird. TEDA, systematisch 1,4-Diazabicyclo(2.2.2)-octan (DABCO) genannt, ist u.a. als Katalysator zur Herstellung von Polyurethanen erforderlich. PA findet z.B. Verwendung als Ausgangsprodukt zur Herstellung von Pharmazeutika.

Es ist bekannt, TEDA und PA durch Reaktion von einfachen Aminvorprodukten, wie Ethanolamin, an heterogenen Katalysatoren herzustellen. Nach WO 87/03592 kann TEDA aus verschiedenen Aminvorprodukten durch Umsetzung an Zeolithen des Pentasil-Typs erhalten werden. Geeignet hierfür sind gemäß WO 87/03592 saure Vertreter der Pentasile, also solche, die in der H$^+$-Form vorliegen.

Auch EP 0 313 753 A2 setzt für solche Umsetzungen Zeolithe des Pentasil-Typs ein. In der allgemeinen Formel werden undifferenziert auch Zeolithe mit verschiedenen Metallkationen angegeben; tatsächlich wirksam sind jedoch gemäß den Ausführungsbeispielen dieser EP-Anmeldung Zeolithe in der H$^+$-Form, wie sie aus DE-OS 32 39 054 bekannt sind.

In EP 312 734 A1 ist die Umwandlung von Piperazin zu Triethylendiamin an Pentasil-Zeolithen beschrieben.

Die in den genannten Schriften offenbarten Befunde entsprechen durchaus dem gängigen Kenntnisstand, demzufolge die Umsetzung der genannten Aminvorprodukte an sauren Katalysatoren vorgenommen werden muß. In völliger Übereinstimmung hiermit zeigen auch die Beispiele von WO 87/03592 in der Reihenfolge H-[Al]ZSM 5 (Beispiel 1), H-[Ga]ZSM 5 (Beispiel 6), H-[Fe]ZSM 5 (Beispiel 8) und H-[B]ZSM 5 (Beispiel 7) deutlich fallende Umsätze und Ausbeuten an TEDA und PA. Zwischen dem Zeolith H- [Al]ZSM 5 einerseits und denen, bei denen Al isomorph durch Ga bzw. B ersetzt ist, gibt es hierbei eine Abstufung bezüglich Umsatz und Ausbeute, und es gibt eine weitere deutliche Abstufung zu negativem Umsatz und Ausbeute, wenn ein solcher isomorpher Austausch von Al durch Fe vorgenommen wurde. Eine solche Abstufung konnte vom Fachmann auch erwartet werden, da in der angegebenen Reihenfolge laut J. Phys. Chem. 89 (1985), 1569-1571 die Säurestärke dieser Zeolithe abfällt. In Übereinstimmung mit diesem Befund zeigen die Vergleichsbeispiele 1 und 2 von WO 87/03592 bei Verwendung von Na-ZSM 5 einen nahe bei Null liegenden Umsatz, was ebenfalls auf eine Säurekatalyse für diese Umwandlung hinweist.

Die Nacharbeitung der in WO 87/03592 und EP 0 313 753 A2 beschriebenen Verfahren, für die in diesen Schriften keine Zeitangaben für Probenahmen und Laufzeit der Reaktionen gemacht werden, ergab nur anfänglich gute Umsätze und Ausbeute, die infolge der Desaktivierung der sauren Zeolithe jedoch sehr bald auf technisch uninteressante Werte zurückgehen.

Entgegen den geschilderten Befunden, denen zufolge bei größerer Säurestärke der Zeolith-Katalysatoren günstigere Werte erhalten werden, wurde nun überraschend gefunden, daß im Gegensatz hierzu beim Einsatz von Zeolithen des Pentasil-Typs mit abgeschwächter Acidität und gleichzeitigem Einsatz von EDA verbesserte Selektivitäten zu den Wertprodukten TEDA und PA gefunden werden und das solche erfindungsgemäß einsetzbaren Zeolithe in ihrer Standzeit die sauren Zeolithe um Größenordnungen übertreffen und nur geringe Mengen an Crackprodukten liefern, wobei gleichzeitig hohe Umsätze erzielt werden. Solche erfindungsgemäß einsetzbaren Zeolithe des Pentasil-Typs mit abgeschwächter Acidität sind Pentasil-Typen, die Alkaliionen enthalten oder bei denen das Aluminium des Zeolithgerüsts isomorph durch Eisen ersetzt ist.

Es wurde ein Verfahren zur Herstellung von Triethylendiamin und Piperazin aus Ethylendiamin an Zeolithen des Pentasil-Typs als Katalysator gefunden, das dadurch gekennzeichnet ist, das man bei einer Temperatur von 270-420°C und bei einer Katalysatorbelastung WHSV (Weight Hourly Space Velocity) von 0,03 bis 2,0 kg Ethylendiamin/kg Katalysator/h in Form von Ethylendiamin-Wasser-Gemischen mit 2-25 Mol Wasser pro Mol Ethylendiamin an einem Pentasil-Zeolith, der Alkaliionen enthält oder bei dem das Aluminium des Zeolithgerüsts isomorph durch Eisen ersetzt ist, umsetzt, wobei Ethylendiamin zu 2-60 % seiner Menge durch rückgeführtes Piperazin ersetzt werden kann.

Zeolithe können allgemein durch die Formel

$$M^1_m[mM^2O_2 \cdot nSiO_2] \cdot qH_2O,$$

in der

$M^2$  ein dreiwertiges Element bedeutet, das gemeinsam mit dem Si das oxidische Gerüst bildet,

$n/m$  das $SiO_2/M^2O_2$-Verhältnis bedeutet,

$M^1$  für ein Äquivalent eines austauschbaren Kations steht, dessen Anzahl Äquivalente dem Anteil m von $M^2$ entspricht und

$q$  die Menge des absorbierten Wassers angibt.

Zeolithe sind dem Fachmann bekannt und zusammenfassend beschrieben in der Monographie D.W. Breck: Zeolite Molecular Sieves, John Wiley & Sons Inc., New York 1974; weitere Publikationen, die insbesondere auf die erfindungsgemäß einsetzbaren Zeolithe von Pentasil-Typ abstellen, sind: Russ. J, Phys. Chem. 55 (1981), 1175; EP 18 090, EP 34 727, EP 54 286, EP 57 016, EP 65 401, EP 113 116, DE-OS 25 48 697, DE-OS 26 43 929, US 3.702.886, US 3.709.979, GB 1.334.243, Zeolites 3 (1983), 155-162 und Zeolites 7 (1987), 398-403.

In einer Variante des erfindungsgemäßen Verfahrens werden Zeolithe eingesetzt, in denen $M^2$ Aluminium ist und $M^1$ zu mindestens 50 % der Menge aller austauschbaren Kationen ein Alkalimetall ist. Das Verhältnis n/m beträgt hierin 10-1000, bevorzugt 10-500, besonders bevorzugt 15-300. Solche Zeolithtypen werden als Pentasile bezeichnet.

In bevorzugter Weise werden unter den Pentasil-Typen die Zeolithstrukturen ZSM 5, ZSM 11, ZSM 8, ZSM 5/ZSM 11-Intermediates, Zeta 1, Zeta 3, ZBM 10, Ultrasil, Ultrazel, TZ-01, NU 4, NU 5, AZ 1 oder ein Gemisch von ihnen eingesetzt. Besonders bevorzugt wird ein Pentasil-Zeolith aus der Gruppe ZSM 5, ZSM 8, ZSM 11, ZSM 5/-ZSM 11-Intermediates oder einem Gemisch von ihnen eingesetzt. Ganz besonders bevorzugt wird ZSM 5 eingesetzt.

Solche erfindungsgemäß einsetzbaren Pentasil-Zeolithe haben einen Gehalt an Alkaliionen von 50-100 % aller austauschbaren Kationen, bevorzugt 80-100 %, besonders bevorzugt 90 bis 100 %. Die Alkaliionen sind solche von Li, Na, K, Rb oder Cs, bevorzugt die von Na, K, Rb oder Cs, besonders bevorzugt die von K, Rb oder Cs, ganz besonders bevorzugt die von K.

In einer anderen Variante des erfindungsgemäßen Verfahrens werden Pentasil-Zeolithe eingesetzt, in denen das Aluminium isomorph durch Eisen ersetzt ist und demzufolge $M^2$ in der obigen Formel für Eisen steht. In solchen Eisen-Pentasilen ist $M^1$ Wasserstoff. Das Verhältnis Si/Fe beträgt 10-100, bevorzugt 12-90, besonders bevorzugt 15-80, Die Herstellung solcher Eisen-Pentasile kann beispielsweise nach J. Chem. Soc., Farad. Trans. 1, Vol. 83 (1987), 487-494 erfolgen.

Das erfindungsgemäße Verfahren wird in der Gasphase durchgefuhrt, wobei der Zeolith-Katalysator im Festbett oder im Wirbelbett vorliegen kann. Für die Durchführung mit im Festbett angeordnetem Katalysator wird der Zeolith in Granulat- oder Kugelform eingesetzt, wozu die Pentasil-Katalysatoren mit einem Binder, wie Aluminiumoxid, Siliciumdioxid oder Tonerden, wie Bentoniten und Montmorilloniten, im Gewichtsverhältnis Pentasil/Binder = 2-5/1 vermischt werden und in bekannter Technik zu solchen Granulaten oder Kugeln verarbeitet werden. Die Korngrößen betragen bevorzugt 0,2-8 mm ø, besonders bevorzugt 0,5-6 mm ø.

Für den Einsatz im Wirbelbett eignet sich eine feinkörnige bis pulverige Struktur mit Korngrößen von 50 bis 500 µm.

Das Ausgangsmaterial für das erfindungsgemäße Verfahren ist EDA selbst oder seine wäßrigen Lösungen. Solche Ausgangsstoffe werden, falls erforderlich, zum erfindungsgemäßen Einsatz so mit Wasser gemischt, daß 2-25 Mol Wasser pro Mol EDA vorliegen. Es ist erfindungsgemäß weiterhin möglich, das EDA mindestens teilweise durch Piperazin zu ersetzen. Dies hat dann eine große technische Bedeutung, wenn der Bedarf an TEDA wesentlich über dem an PA liegt und so das Koppelprodukt PA weiter in das stärker gefragte TEDA umgewandelt werden kann, Wenngleich der 100 %ige Ersatz von Ethylendiamin durch Piperazin in der technischen Praxis selten sein wird, kommen doch bei Rückführungen von PA ein Ersatz von 2-60 %, bevorzugt 5-40 % des Ethylendiamins durch Piperazin in Betracht. Es ist weiterhin möglich, zusätzlich zum Wasser Inertstoffe zuzusetzen, wie Wasserstoff, Stickstoff, Edelgase, wie Argon, $NH_3$ oder Kohlenwasserstoffe, wie Methan, Butan, Pentan, Cyclohexan, Toluol oder andere. Sollte das Einsatzmaterial Ethylendiamin oder sein Substitut Piperazin in der Lösung eines solchen Kohlenwasserstoffes zur Verfügung stehen, kann demzufolge auch eine solche Lösung eingesetzt werden, wenn zusätzlich Wasser in der oben beschriebenen Menge eingesetzt wird.

Es hat sich als vorteilhaft erwiesen, in der Variante des erfindungsgemäßen Verfahrens, in der Alkaliionen enthaltender Pentasil eingesetzt wird, Wasser in einer bevorzugten Menge von 4-20 Mol, besonders bevorzugt 5-15 Mol pro Mol Ethylendiamin bzw, Piperazin einzusetzen, In der Variante des erfindungsgemäßen Verfahrens, in der ein Eisen-Pentasil eingesetzt wird, werden bevorzugt 5-25 Mol Wasser, besonders bevorzugt 6-15 Mol Wasser, ganz besonders bevorzugt 6-11 Mol Wasser pro Mol Ethylendiamin eingesetzt.

Die Reaktionstemperatur für das erfindungsgemäße Verfahren liegt bei 270-420°C, bevorzugt bei 280-410°C, besonders bevorzugt bei 300-400°C. Das erfindungsgemäße Verfahren kann unter einem Druck von 0,01-40 bar, bevorzugt 0,05-35 bar, besonders bevorzugt 0,1-30 bar durchgeführt werden. Die Katalysatorbelastung wird ausgedrückt als WHSV (Weight Hourly Space Velocity) in kg EDA bzw. PA pro kg Pentasil-Katalysator pro Stunde und kann Werte von 0,03-2,0, bevorzugt 0,05-1,8, besonders bevorzugt 0,1-1,5, annehmen.

Es wurde weiterhin gefunden und stellt eine interessante Bereicherung des erfindungsgemäßen Verfahrens dar, daß durch die Wahl der Reaktionsbedingungen das Verhältnis von TEDA zu PA variabel gestaltet werden kann, so das das erfindungsgemäße Verfahren dem Bedarf an diesen beiden Wertstoffen flexibel angepaßt werden kann. So kann man beispielsweise bei sonst gleichen Bedingungen und bei fallender Katalysatorbelastung eine Umkehrung des Verhältnisses TEDA/PA von beispielsweise 3/6 auf ca. 6/4 erreichen, ohne das die Gesamtselektivität für diese beiden Wertstoffe nennenswert verändert wird. Ähnliche Effekte erzielt man bei sonst gleichen Bedingungen durch Temperaturänderung, wobei steigende Temperaturen den TEDA-Anteil des Produktgemisches erhöhen. Eine Feineinstellung und Optimierung kann beispielsweise durch Anpassung beider genannter Parameter erfolgen. Diese Umkehr des TEDA/PA-Verhältnisses ist besonders ausgeprägt für Eisen-Pentasil-Katalysatoren.

Beispiel 1

Synthese und Modifizierung der ZSM 5-Silikate

ZSM 5 ($SiO_2/Al_2O_3$ = 40)

Die Synthese der Alkalipentasile (ZSM 5) für die folgenden Beispiele wurde nach US-PS 3 702 886 und Zeolites 3, S. 155-162 (1983) durchgeführt:

2 g $Al(NO_3)_3 \cdot 9 H_2O$ und 4,6 g Tetrapropylammoniumbromid wurden in 32 ml Wasser gelöst. Hierzu wurden nacheinander unter Rühren 32 g Kieselsäure-Sol (40 Gew.-% $SiO_2$ in Wasser, Handelsprodukt Ludox AS 40 der Fa. DuPont) und 43,6 ml Ammoniumhydroxid-Lösung (25 Gew.-% in Wasser) gegeben. Die Mischung wurde unter Rotation im Edelstahlautoklaven viereinhalb Tage lang bei 180°C gehalten. Das weiße Kristallisat wurde bei 120°C getrocknet und anschließend in Luft 16 h lang bei 550°C kalziniert.

So synthetisierte und kalzinierte ZSM 5-Zeolithe wurden durch zweimaligen Ionenaustausch mit einem 50-fachen Gewichtsüberschuß einer 1 n-Alkalisalzlosung, wie NaCl-, KCl-, RbCl- oder CsCl-Lösung, acht Stunden bei ca. 80°C behandelt, gründlich gewaschen und bei 120°C getrocknet.

Zur Überführung der Syntheseform der Na-ZSM 5-Zeolithe in die korrespondierende saure H-Form wurde zweimal mit einem 50-fachen Überschuß einer 1 n $NH_4Cl$-Lösung bei 80°C 8 h behandelt, getrocknet und bei 450°C 4 h lang im $N_2$-Strom kalziniert.

Katalysator A =  K ZSM 5
Katalysator B =  Rb ZSM 5
Katalysator C =  Cs ZSM 5
Katalysator D =  H ZSM 5
Katalysator E =  Na ZSM 5
Katalysator F =  Li ZSM 5

Vor Beginn der Reaktion wurde der jeweilige Zeolith im Reaktionsrohr 4 h lang bei 450°C im $N_2$-Strom erhitzt.

Beispiele 2 bis 19

Umsetzung von Ethylendiamin (EDA) und Wasser zu Triethylendiamin (TEDA) und Piperazin (PA)

Die Umsetzungen wurden in einer Strömungsapparatur mit Festbettreaktor unter Atmosphärendruck durchgeführt. Das Ausgangsgemisch wurde in einem Vorverdampfer in die Gasphase gebracht, mit einem leichten Stickstoffstrom weitergeleitet, auf die gewünschte Reaktionstemperatur erhitzt und durchströmte dann nach Eintritt in den Reaktor die Katalysatorschüttung. Über eine Probenahmeeinheit konnten zu beliebigen Zeiten im on-line-Betrieb Gasproben in einen Kapillar-Gaschromatographen mit einem Flammen-Ionisations-Detektor (FID) eingeschleust und analysiert werden. Für eine destillative Aufarbeitung wurde das Reaktionsgemisch auf 0-10°C gekühlt, kondensiert und gesammelt.

EP 0 423 526 B1

**Tabelle 1** (Beispiele 2 und 3):

Vergleich von H-ZSM 5 (Kat D) mit Cs-ZSM 5 (Kat C) bei $340^\circ$ C

$WHSV_{EDA}$ = 0,16 $h^{-1}$, Mol $H_2O$/Mol EDA = 10/1

| Beispiel | Kat | $t_{Reakt}$(h) | $X_{EDA}$% | $S_{TEDA}$% | $S_{PA}$% | SG% | Bemerkungen |
|---|---|---|---|---|---|---|---|
| 2 (Vergleich) | D | 1 | 100 | 41 | 25 | 66 | $X_{EDA}$ sinkt |
|  |  | 10 |  | 47 | 28 | 22 | 50 | weiter ab |
| 3 | C | 10 | 55 | 40 | 55 | 95 | nach wenigen |
|  |  | 14 | 55 | 40 | 55 | 95 | Std. station. |
|  |  |  |  |  |  |  | Verhältnisse |

$t_{Reakt}$(h) = Laufzeit bei der Probenahme

$X_{EDA}$% = Umsatz an EDA

$S_{TEDA}$% = Selektivität bezüglich TEDA

$S_{PA}$% = Selektivität bezüglich PA

SG% = Gesamtselektivität bezüglich PA + TEDA

$WHSV_{EDA}$ = Belastung des Katalysators in g EDA/g Kat./h

**Tabelle 2** (Beispiele 4 bis 7):

Umwandlung von EDA an Na ZSM 5 (Kat E) bei 340°C und $WHSV_{EDA}$ von 0,32 $h^{-1}$ in Abhängigkeit vom Molverhältnis $H_2O$ zu EDA; Probenahme nach 10 h

| Beispiel | Mol-V. $H_2O/EDA$ | $X_{EDA}$% | $S_{TEDA}$% | $S_{PA}$% | SG% | Bemerkungen |
|---|---|---|---|---|---|---|
| 4 | 4/1 | 58 | 27 | 58 | 85 | $X_{EDA}$ konstant |
| 5 | 10/1 | 71 | 35 | 59 | 94 | |
| 6 (Vergleich) | 0/1 | 45 | 16 | 54 | 70 | $X_{EDA}$ sank |
| 7 (Vergleich) | 1/1 | 53 | 20 | 56 | 76 | |

Wie die Beispiele 2 und 3 zeigen, war H-ZSM 5 nach kurzer Laufzeit erheblich desaktiviert und der Umsatz sank weiter, während mit Cs-ZSM 5 nach wenigen Stunden Laufzeit stationäre Verhältnisse und konstante Werte vorlagen und erhalten blieben bei ausgezeichneten Selektivitäten.

Nach den Beispielen 4 und 5 erhielt man mit Alkali-ZSM 5 besonders gute, technisch interessante Umsätze und Selektivitäten, wenn man erfindungsgemäße Verhältnisse von Wasser-EDA-Gemischen einsetzte. Nach 10 h war der Umsatz von EDA in Beispiel 4 oder 5 praktisch konstant. Die Vergleichsbeispiele wiesen eine laufende Desaktivierung auf.

**Tabelle 3** (Beispiele 8 bis 11):

Umwandlung von EDA an Cs-ZSM 5 (Kat C) bei verschiedenen Temperaturen T und Belastungen. Probenahme jeweils nach 10 h bei stationären Verhältnissen

| Beispiel | T ($^{\circ}$C) | WHSV$_{EDA}$ | X$_{EDA}$% | S$_{TEDA}$% | S$_{PA}$% |
|---|---|---|---|---|---|
| 8 | 350 | 0,16 | 56 | 41 | 56 |
| 9 | 360 | 0,32 | 70 | 41 | 47 |
| 10 | 375 | 0,32 | 71 | 44 | 44 |
| 11 | 380 | 0,60 | 55 | 45 | 40 |

Bei Erhöhung der Belastung konnte bei gleichzeitiger Erhöhung der Temperatur ein hoher Umsatz erzielt werden (Beispiele 8, 9, 10). Bei weiterer Erhöhung der Belastung auf 0,6, also wiederum fast den doppelten Wert (Beispiel 11), fiel der Umsatz wieder ab. Die Selektivitäten für die Wertprodukte TEDA und PA betrugen zwischen 85 und 97 %.

**Tabelle 4** (Beispiele 12 bis 16):

Umwandlung von EDA an verschiedenen Alkali-ZSM 5 bei einem Molverhältnis $H_2O$/EDA = 10/1 und einer WHSV$_{EDA}$ = 0,65 h$^{-1}$. Probenahme nach 10 h Laufzeit bei stationären Verhältnissen

| Beispiel | Kat. | T$_{Reakt}$$^{\circ}$C | X$_{EDA}$% | S$_{PA}$% | S$_{TEDA}$% | SG% |
|---|---|---|---|---|---|---|
| 12 | F | 350 | 30 | 59 | 38 | 97 |
| 13 | E | 325 | 31 | 80 | 19 | 99 |
| 14 | A | 322 | 48 | 78 | 21 | 99 |
| 15 | B | 324 | 45 | 76 | 22 | 99 |
| 16 | C | 327 | 42 | 74 | 25 | 99 |

In allen Beispielen 12-16, also an allen erfindungsgemäßen Katalysatoren, wurden fast quantitative Selektivitäten erreicht. Li- und Na-ZSM 5 bewirken jedoch etwas geringere Umsätze als K-, Rb- und Cs-ZSM 5.

**Tabelle 5** (Beispiele 17 bis 19):

Abhängigkeit der $S_{TEDA}$% und $S_{PA}$% vom Umsatz $X_{EDA}$% an Kat A bei einer $WHSV_{EDA}$ von 0,65 h$^{-1}$

| Beispiel | $X_{EDA}$% | $S_{PA}$% | $S_{TEDA}$% |
|----------|------------|-----------|-------------|
| 17 | 40 | 80 | 19 |
| 18 | 60 | 72 | 26 |
| 19 | 90 | 46 | 43 |

Die Flexibilität des erfindungsgemäßen Verfahrens ließ sich aus den obigen Beispielen erkennen. Die Beeinflussung der jeweiligen Selektivitäten für PA und TEDA wurden durch die Veränderung der $WHSV_{EDA}$ und der Temperatur ermöglicht.

Beispiel 20

Mit einer WHSV von 0,30 h$^{-1}$ wurde bei 340°C ein EDA-Wasser-Gemisch von 1/10 (Molverhältnis) über den Kat A geleitet. Nach Einstellung eines stationären Zustands konnte nach 3 Tagen Laufzeit praktisch keine Desaktivierung festgestellt werden. Umsatz ($X_{EDA}$ 82 %) und Selektivitäten ($S_{PA}$ 58 % und $S_{TEDA}$ 38 %) waren nach wie vor ausgezeichnet.

Beispiel 21

Synthese und Modifizierung der $M^{3+}$- ZSM 5- Silikate

Al-ZSM 5 (Si/Al = 40) : Katalysator I

2 g Al(NO$_3$)$_3$ · 9 H$_2$O und 4,6 g Tetrapropylammoniumbromid wurden in 32 ml Wasser gelöst. Hierzu wurden nacheinander unter Rühren 32 g Ludox AS 40 (40 Gew.-% SiO$_2$ in Wasser, DuPont) und 43,6 ml Ammoniumhydroxid-Lösung (25 Gew.-% in Wasser) gegeben. Die Mischung wurde unter Rotation im Edelstahlautoklaven viereinhalb Tage lang bei 180°C gehalten. Das weise Kristallisat wurde bei 120°C getrocknet und anschließend in Luft für 16 h bei 550°C kalziniert. Röntgenbeugungsdiagramme ergaben eine gute Kristallinität und Reinheit.

Zur Überführung in die H-Form wurde der Zeolith einem Ionenaustausch mit einem 50-fachen Überschuß (massenbezogen) einer 1n NH$_4$Cl-Lösung bei 80°C unterworfen. Die Austauschdauer betrug 8 h. Der Zeolith wurde bei 120°C getrocknet.

Zur Überführung in die H$^+$ -Form wurde der Zeolith vor dem Einsatz im Reaktor für 4 h bei 450°C im N$_2$-Strom (3 l/h) gehalten.

Ga-ZSM 5 (Si/Ga= 27): Katalysator II

15,2 g Ga(NO$_3$)$_3$ · 9 H$_2$O wurden in 100 g Wasser gelöst. Die Lösung wurde mit 16 g H$_2$SO$_4$ (96 %) angesäuert, und 200 g Natronwasserglas (28,5 Gew.-% SiO$_2$ in Wasser, Merck) in 200 g Wasser wurden hinzugegeben. Es trat die sofortige Bildung eines schwachgelben Gels ein. In dieses Gel wurde eine Lösung von 24 g Tetrapropylammoniumbromid und 40 g Wasser gegeben. Die Mischung wurde unter Rotation in Edelstahlautoklaven drei Tage lang bei 170°C gehalten. Daß weiße Kristallisat wurde bei 120°C getrocknet und anschließend in Luft für 16 h bei 480°C kalziniert. Röntgenbeugungsdiagramme des weißen Pulvers ergaben eine gute Kristallinität und Reinheit. Die weiteren Operationen zur Erzeugung des einsatzfähigen Galliumsilicats wurden analog dem Al-HZSM-5 durchgeführt.

Fe-ZSM-5 (Si/Fe = 38): Katalysator III

In eine Lösung aus 37,8 g Natronwasserglas (28,5 Gew.-% SiO$_2$ in Wasser, Merck), 4,3 g Tetrapropylammoniumbromid und 50 g Wasser wurde unter Rühren eine Lösung von 1,02 g Fe$_2$(SO$_4$)$_3$ · 7 H$_2$O in 40 g Wasser gegeben. Anschließend wurde mit verdünnter $H_2SO_4$ ein pH-Wert von 11 eingestellt. Die dunkelblaue, hochviskose Lösung wurde zunächst 30 min. bei Raumtemperatur gerührt, und dann ließ man für 16 h im Edelstahlautoklaven bei 160°C statisch kristallisieren. Das schwachgelb gefärbte Kristallisat wurde bei 120°C getrocknet und anschließend in Luft für 16 h bei 480°C kalziniert. Röntgenbeugungsdiagramme des schwachgelben Pulvers ergaben eine gute Kristallinität und Reinheit.

Das Si/Fe-Verhältnis konnte durch entsprechende Änderungen des Anteils an $Fe_2(SO_4)_3 \cdot 7\ H_2O$ im Syntheseansatz variiert werden. Die weiteren Operationen zur Erzeugung des einsatzfähigen Eisensilikats wurden analog dem Al-HZSM 5 durchgeführt.

Fe-HZSM 5 (Si/Fe = 20):      Katalysator IV
Fe-HZSM 5 (Si/Fe = 60):      Katalysator V
Beide hergestellt analog III.


Beispiele 22 bis 33

Umsetzung von Ethylendiamin (EDA) und Wasser zu Triethylendiamin (TEDA) und Piperazin (PA)


Durchführung der Reaktion, allgemeine Beschreibung

Die katalytischen Experimente wurden in einer Strömungsapparatur mit Festbettreaktoren unter Atmosphärendruck (Ausnahme Beispiele 39 bis 44) durchgeführt. Das Ausgangsgemisch wurde in einen Vorverdampfer in die Gasphase gebracht, mit einem leichten Stickstoffstrom weitergeführt, auf die gewünschte Reaktionstemperatur erhitzt und in den Reaktor eingeleitet, in dem der im Festbett angeordnete Katalysator von dem Gas durchströmt wurde. Über eine Probenahmeeinheit konnten zu beliebigen Zeiten in on-line-Betrieb Gasproben in einen Kapillar-Gaschromatographen mit FID als Detektor eingeschleust und analysiert werden. Für eine destillative Aufarbeitung wurde das Reaktionsprodukt abgekühlt auf 0 bis 10°C, kondensiert und gesammelt.

Tabelle 6 zeigt deutlich folgende Zusammenhänge:
- Al-HZSM 5 war schon nach 2 h Laufzeit desaktiviert und der Umsatz fiel auf 24 bzw. 32 % ab, die Menge der Crackprodukte lag mit 13 % relativ hoch. Die Selektivität an TEDA und PA betrug 41 bis 68 %.
- Fe-HZSM 5 bewirkte selbst nach 15 h einen fast dreifach höheren Umsatz als Al-ZSM 5 und eine Selektivität, bezogen auf TEDA und PA, von 84 bis 92 %.

EP 0 423 526 B1

Tabelle 6 (Beispiele 22 bis 27):

Vergleich von Al-HZSM 5 (Kat. I) mit Fe-HZSM 5 (Kat. III) bei der Umsetzung von
Ethylendiamin (EDA) bei 340°C und einer WHSV an Ethylendiamin von 0,32 $h^{-1}$

| Bsp. | Kat | $\dfrac{H_2O}{EDA}$ | $t_{Reakt.}$(h) | $X_{EDA}$,% | $S_{crack.}$,% | $S_{PA}$% | $S_{TEDA}$% | SG% |
|---|---|---|---|---|---|---|---|---|
| 22 (Vergleich) | III | 1 | 15 | 74 | 5,0 | 37 | 47 | 84 |
| 23 | III | 4 | 15 | 77 | 4,4 | 39 | 49 | 88 |
| 24 | III | 10 | 15 | 83 | 3,5 | 42 | 50 | 92 |
| 25 (Vergleich) | I | 1 | 2 | 24 | 12 | 35 | 6 | 41 |
| 26 (Vergleich) | I | 4 | 2 | 36 | 13 | 30 | 15 | 45 |
| 27 (Vergleich) | I | 10 | 2 | 32 | 10 | 42 | 26 | 68 |

WHSV = Katalysatorbelastung (g Reaktand pro g Kat. pro Stunde)

$$\frac{H_2O}{EDA} = \frac{Mol\ H_2O}{Mol\ EDA} \ ; \ t_{Reakt.}h = \text{Reaktionszeit in h, zu der die Probenahme erfolgt}$$

S = Selektivität, bezogen auf umgesetztes EDA

$S_{crack}$ = betrifft undefinierte Zersetzungsprodukte

$X_{EDA}$ = umgesetztes EDA

SG = Selektivität an TEDA + PA, bezogen auf umgesetztes EDA

$S_{PA}$% = Selektivität bez. PA

$S_{TEDA}$% = Selektivität bez. TEDA

<u>Tabelle 7</u> (Beispiele 28 bis 30, alles Vergleichsbei-
spiele)

Vergleich der Katalysatoren I, II und III bei der Umwandlung von Ethanolamin (EA) bei 340° C,

Mol $H_2O$/Mol EA = 10/1

$WHSV_{EA}$ = 0,1 $h^{-1}$

| Bsp. | Kat. | $t_{Reakt}$(h) | $X_{EA}$% | $t_{Reakt}$(h) | $X_{EA}$% |
|------|------|------|------|------|------|
| 28 | I | 1 | 100 | 2,0 | 60 |
| 29 | II | 1 | 100 | 2,3 | 60 |
| 30 | III | 1 | 100 | 4,0 | 70 |

Beim Einsatz von Ethanolamin-Wasser-Gemischen wurden alle Pentasile Al-, Ga- und Fe-HZSM 5 rasch desaktiviert, denn der Umsatz fiel in wenigen Stunden von 100 % auf technisch uninteressante Werte. Hier unterschied sich Fe-Pentasil nur wenig von Al-Pentasil, die Selektivitäten für TEDA lagen in beiden Fällen etwa gleich hoch.

<u>Tabelle 8</u> (Beispiele 31 bis 33):

Umsetzung von EDA an den Katalysatoren I, II und III bei 340°C, $WHSV_{EDA}$ = 0,16 $h^{-1}$ und Mol $H_2O$/Mol EDA = 10/1

| Bsp. | Kat | $t_{Reakt}$(h) | $X_{EDA}$% | $S_{TEDA}$% | $S_{PA}$% | %SG | Bemerkung |
|------|-----|------|------|------|------|------|-----------|
| 31 | I | 1 | 100 | 41 | 25 | 66 | $X_{EDA}$% |
| (Vgl) | I | 4 | 65 | 28 | 23 | 51 | sinkt |
| | I | 10 | 47 | 28 | 22 | 55 | weiter |
| | I | 20 | 35 | 25 | 22 | 47 | |
| 32 | II | 1 | 100 | 20 | - | 20 | $X_{EDA}$% |
| (Vgl) | II | 4 | 100 | 45 | 28 | 73 | sinkt |
| | II | 10 | 96 | 44 | 30 | 74 | weiter |
| | II | 20 | 60 | 39 | 35 | 74 | |
| 33 | III | 1 | 100 | 30 | 10 | 40 | $X_{EDA}$% |
| | III | 4 | 100 | 55 | 30 | 85 | konstant; |
| | III | 10 | 100 | 48 | 42 | 90 | $S_{TEDA}$% und |
| | III | 20 | 100 | 48 | 42 | 90 | $S_{PA}$% |
| | | | | | | | nach 10 h |
| | | | | | | | konstant |

Zu Tabelle 8:

Im Vergleich mit Tabelle 7 erhielt man andere Ergebnisse, wenn man von EDA ausging. Hier wurde sofort nach Beginn ein drastischer Aktivitätsabfall für Al-HZSM 5, nach 10 h ein weniger steiler für Ga-HZSM 5, jedoch auch nach 20 h keine Aktivitätsminderung für Fe-HZSM 5 beobachtet.

Beispiel 34 (Tabelle 9):

**Tabelle 9**

**Langzeitversuch mit Katalysator IV bei 325°C, einem Molverhältnis $H_2O$/EDA von 10/1 und einer $WHSV_{EDA}$ von 0,30 $h^{-1}$**

| $t_{Reakt}(h)$ | $X_{EA}\%$ | $S_{TEDA}\%$ | $S_{PA}\%$ | SG % |
|---|---|---|---|---|
| 8 | 71 | 40 | 53 | 93 |
| 20 | 69 | 39 | 56 | 95 |
| 40 | 68 | 38 | 57 | 95 |
| 60 | 67 | 38 | 57 | 95 |
| 80 | 67 | 38 | 57 | 95 |

Nach anfänglichen geringfügigen Veränderungen stellten sich bald konstante Umsatz- und Selektivitätswerte auf hohem Niveau ein.

Beispiele 35 bis 38 (Tabelle 10)

**Tabelle 10**

**Abhängigkeit des Verhältnisses von PA zu TEDA von der $WHSV_{EDA}(h^{-1})$ bei 340°C,**

**Mol $H_2O$/Mol EDA = 10/1**

**am Katalysator IV**

| Bsp. | $WHSV_{EDA}(h^{-1})$ | $X_{EDA}\%$ | $S_{PA}\%$ | $S_{TEDA}\%$ | SG% |
|---|---|---|---|---|---|
| 35 | 0,11 | 100 | 30 | 62 | 92 |
| 36 | 0,16 | 100(86) | 40(41) | 51(47) | 91(89) |
| 37 | 0,32 | 83(68) | 43(46) | 51(51) | 95(97) |
| 38 | 0,65 | 64 | 57 | 41 | 98 |

Eine Erhöhung der Katalysatorbelastung mit EDA von 0,11 auf 0,65 bewirkte eine merkliche Verschiebung von TEDA zu PA im Reaktionsprodukt, wobei sogar eine gewisse Erhöhung der Selektivität erreicht wurde. Die Werte in Klammern gelten für Katalysator V und weisen die gleiche Tendenz auf.

Beispiele 39 bis 44 (Tabelle 11)

## Tabelle 11

Durchführung der Umsetzung von EDA/$H_2O$ = 1/10 unter erhöhtem Druck bei verschiedenen Belastungen und Temperaturen im $N_2$-Strom über Kat. III

| Bsp. | T (°C) | P (bar) | $N_2$ (l/h) | WHSV EDA | $X_{EDA}$ % | $S_{PA}$ % | $S_{TEDA}$ % | SG % |
|------|--------|---------|-------------|----------|-------------|------------|--------------|------|
| 39 | 340 | 10 | 5 | 0,23 | 96 | 26 | 65 | 91 |
| 40 | 340 | 10 | 5 | 0,65 | 75 | 37 | 56 | 93 |
| 41 | 330 | 30 | 14 | 0,65 | 81 | 35 | 57 | 92 |
| 42 | 330 | 30 | 14 | 0,97 | 68 | 39 | 51 | 90 |
| 43 | 370 | 3 | 14 | 0,65 | 83 | 36 | 55 | 91 |
| 44 | 370 | 50 | 14 | 0,58 | 100 | 21 | 65 | 86 |

$N_2$ l/h: Volumenfluß von $N_2$ bei Normaldruck

Die Beispiele zeigen, das sich durch Anwendung von Druck, Temperatur und Katalysatorbelastung die Umsätze und die Selektivitäten für PA und TEDA verschieben ließen, ohne das die Gesamtselektivität für die beiden Wertprodukte nennenswert verändert wurde.

**Patentansprüche**

1.  Verfahren zur Herstellung von Triethylendiamin und Piperazin aus Ethylendiamin an Zeolithen des Pentasil-Typs als Katalysator, dadurch gekennzeichnet, daß man bei einer Temperatur von 270-420°C und bei einer Katalysatorbelastung WHSV (Weight Hourly Space Velocity) von 0,03-2,0 kg Ethylendiamin/kg Katalysator/h in Form von Ethylendiamin-WasserGemischen mit 2-25 Mol Wasser pro Mol Ethylendiamin an einem Pentasil-Zeolith, der Alkaliionen enthält oder bei dem das Aluminium des Zeolithgerüsts isomorph durch Eisen ersetzt ist, umsetzt, wobei Ethylendiamin zu 2-60 % seiner Menge durch rückgeführtes Piperazin ersetzt werden kann.

2.  Verfahren nach Anspruch 1, dadurch gekennzeichnet, daß man als Alkaliionen enthaltenden Pentasil-Zeolith einen aus der Gruppe ZSM 5, ZSM 11, ZSM 8, ZSM 5/ZSM 11-Intermediates, Zeta 1, Zeta 3, ZBM 10, Ultrasil, Ultrazel, TZ-01, NU 4, NU 5, AZ 1 oder einem Gemisch von ihnen, bevorzugt einer aus der Gruppe ZSM 5, ZSM 8, ZSM 11, ZSM 5/ZSM 11-Intermediates oder einem Gemisch von ihnen, besonders bevorzugt ZSM 5 mit einem Gehalt an Alkaliionen von 50-100 % aller austauschbarer Kationen, bevorzugt 80-100 % aller austauschbarer Kationen, besonders bevorzugt 90-100 % aller austauschbarer Kationen, einsetzt, wobei die Alkaliionen die von Li, Na, K, Rb oder Cs, bevorzugt die von Na, K, Rb oder Cs, besonders bevorzugt die von K, Rb oder Cs, ganz besonders bevorzugt die von K sind.

3.  Verfahren nach Anspruch 2, dadurch gekennzeichnet, daß das $SiO_2/Al_2O_3$-Verhältnis 10-1000, bevorzugt 10-500, besonders bevorzugt 15-300 beträgt.

4.  Verfahren nach Anspruch 1, dadurch gekennzeichnet, daß als Eisen-Pentasil ein solcher eingesetzt wird, bei dem das Verhaltnis Si/Fe = 10-100, bevorzugt = 12-90, besonders bevorzugt = 15-80 ist.

**5.** Verfahren nach Anspruch 1, dadurch gekennzeichnet, daß das Ethylendiamin zu 5-40 % seiner Menge durch Piperazin ersetzt wird.

**6.** Verfahren nach Anspruch 1, dadurch gekennzeichnet, daß zusatzlich zum Wasser Inertstoffe, wie $H_2$, $N_2$, Edelgase, $NH_3$ oder Kohlenwasserstoffe, eingesetzt werden.

**7.** Verfahren nach Anspruch 2, dadurch gekennzeichnet, daß 4-20 Mol Wasser, bevorzugt 5-15 Mol Wasser pro Mol Ethylendiamin eingesetzt werden.

**8.** Verfahren nach Anspruch 4, dadurch gekennzeichnet, daß 5-25 Mol Wasser, bevorzugt 6-15 Mol Wasser, besonders bevorzugt 6-11 Mol Wasser pro Mol Ethylendiamin eingesetzt werden.

**9.** Verfahren nach Anspruch 1, dadurch gekennzeichnet, daß zur Steigerung des Verhältnisses Triethylendiamin zu Piperazin im Reaktionsprodukt niedrigere Werte der Katalysatorbelastung, höhere Reaktionstemperaturen oder eine Kombination von niedriger Katalysatorbelastung und höherer Temperatur angewandt werden.

## Claims

**1.** Process for the preparation of triethylenediamine and piperazine from ethylenediamine over zeolites of the pentasil type as catalyst, characterized in that the reaction is carried out at a temperature of 270-420°C and at a weight hourly space velocity (WHSV) of 0.03 to 2.0 kg of ethylenediamine/kg of catalyst/h over a pentasil zeolite which contains alkali metal ions or in which the aluminium of the zeolite skeleton has been isomorphously replaced by iron, the feedstock being in the form of ethylenediamine-water mixtures, containing 2-25 mol of water per mole of ethylenediamine, in which 2-60% of the ethylenediamine may be replaced by recirculated piperazine.

**2.** Process according to Claim 1, characterized in that the alkali metal ion-containing pentasil zeolite used is one from ZSM 5, ZSM 11, ZSM 8, ZSM 5/ZSM 11-intermediates, Zeta 1, Zeta 3, ZBM 10, Ultrasil, Ultrazel, TZ-01, NU 4, NU 5, AZ 1 or a mixture thereof, preferably one from ZSM 5, ZSM 8, ZSM 11, ZSM 5/ZSM 11-intermediates or a mixture thereof, particularly preferably ZSM 5 having an alkali metal ion content of 50-100% of all exchangeable cations, preferably 80-100% of all exchangeable cations, particularly preferably 90-100% of all exchangeable cations, the alkali metal ions being those of Li, Na, K, Rb or Cs, preferably those of Na, K, Rb or Cs, particularly preferably those of K, Rb or Cs, most particularly preferably that of K.

**3.** Process according to Claim 2, characterized in that the $SiO_2/Al_2O_3$ ratio is 10-1,000, preferably 10-500, particularly preferably 15-300.

**4.** Process according to Claim 1, characterized in that the iron-pentasil used is one in which the ratio Si/Fe = 10-100, preferably = 12-90, particularly preferably = 15-80.

**5.** Process according to Claim 1, characterized in that 5-40% of the ethylenediamine is replaced by piperazine.

**6.** Process according to Claim 1, characterized in that, in addition to the water, inert substances such as $H_2$, $N_2$, inert gases, $NH_3$ or hydrocarbons are used in the feed.

**7.** Process according to Claim 2, characterized in that 4-20 mol of water, preferably 5-15 mol of water are used per mole of ethylenediamine.

**8.** Process according to Claim 4, characterized in that 5-25 mole of water, preferably 6-15 mol of water, particularly preferably 6-11 mol of water are used per mole of ethylenediamine.

**9.** Process according to Claim 1, characterized in that the ratio of triethylenediamine to piperazine in the reaction product is increased using lower space velocities, higher reaction temperatures or a combination of low space velocity and higher temperature.

**Revendications**

1. Procédé de préparation de la triéthylènediamine et de la pipérazine à partir de l'éthylènediamine sur des catalyseurs consistant en zéolites du type pentasil, caractérisé en ce que l'on fait réagir à une température de 270 à 420°C et une charge de catalyseur VSHP (vitesse spatiale horaire en poids) de 0,03 à 2,0 kg d'éthylènediamine par kilogramme de catalyseur et par heure, sous la forme de mélanges éthylènediamine-eau contenant 2 à 25 mol d'eau par mole d'éthylènediamine, sur une zéolite du type pentasil contenant des ions alcalins ou dans laquelle l'aluminium du squelette de la zéolite a subi un remplacement isomorphe par le fer, l'éthylènediamine pouvant être remplacée en proportions de 2 à 60 % de son poids par de la pipérazine recyclée.

2. Procédé selon la revendication 1, caractérisé en ce que l'on utilise en tant que zéolites du type pentasil contenant des ions alcalins une zéolite du groupe ZSM 5, ZSM 11, ZSM 8, intermédiaires ZSM 5/ZSM 11, Zêta 1, Zêta 3, ZSM 10, Ultrasil, Ultrazel, TZ-01, NU 4, NU 5, AZ 1 ou leurs mélanges, de préférence du groupe ZSM 5, ZSM 8, ZSM 11, intermédiaires ZSM 5/ZSM 11 ou leurs mélanges, tout spécialement ZSM 5, à une teneur en ions alcalins de 50 à 100 % de tous les cations échangeables, plus spécialement de 80 à 100 % de tous les cations échangeables et mieux encore de 90 à 100 % de tous les cations échangeables, les cations alcalins étant ceux de Li, Na, K, Rb ou Cs, de préférence de Na, K, Rb ou Cs, et tout spécialement de K, Rb ou Cs et dans les meilleures conditions de K.

3. Procédé selon la revendication 2, caractérisé en ce que le rapport $SiO_2/Al_2O_3$ est de 10 à 1 000, de préférence de 10 à 500 et tout spécialement de 15 à 300.

4. Procédé selon la revendication 1, caractérisé en ce que l'on utilise en tant que pentasil ferreux un pentasil dans lequel le rapport Si/Fe est de 10 à 100, de préférence de 12 à 90 et tout spécialement de 15 à 80.

5. Procédé selon la revendication 1, caractérisé en ce que l'on remplace l'éthylènediamine pour 5 à 40 % de son poids par de la pipérazine.

6. Procédé selon la revendication 1, caractérisé en ce que l'on met en oeuvre, en plus de l'eau, des substances inertes telles que $H_2$, $N_2$, des gaz nobles, $NH_3$ ou des hydrocarbures.

7. Procédé selon la revendication 2, caractérisé en ce que l'on met en oeuvre de 4 à 20 mol d'eau, de préférence de 5 à 15 mol d'eau par mole d'éthylènediamine.

8. Procédé selon la revendication 4, caractérisé en ce que l'on utilise de 5 à 25 mol d'eau, de préférence de 6 à 15 mol d'eau et tout spécialement de 6 à 11 mol d'eau par mole d'éthylènediamine.

9. Procédé selon la revendication 1, caractérisé en ce que, pour accroître les rapports triéthylènediamine/pipérazine dans le produit de réaction, on opère à une charge plus faible du catalyseur, à une température de réaction plus élevée, ou en combinant une charge plus faible du catalyseur et une température plus élevée.